# EUROPÄISCHE PATENTSCHRIFT

(11) **EP 2 750 673 B1**
(45) Veröffentlichungstag und Bekanntmachung des Hinweises auf die Patenterteilung: **09.08.2017**
(21) Anmeldenummer: 12750597.2
(22) Anmeldetag: 24.08.2012
(51) Int. Cl.: A61K 9/70, A61K 31/197

(54) **TRANSDERMALES THERAPEUTISCHES SYSTEM FÜR 5-AMINOLÄVULINSÄUREHYDROCHLORID**
TRANSDERMAL THERAPEUTIC SYSTEM FOR 5-AMINOLEVULINIC ACID HYDROCHLORIDE
SYSTÈME THÉRAPEUTIQUE TRANSDERMIQUE POUR LE CHLORHYDRATE D'ACIDE 5-AMINOLÉVULINIQUE

(30) Priorität: 31.08.2011 DE 102011111865
(43) Veröffentlichungstag der Anmeldung: 09.07.2014
(73) Patentinhaber: LTS LOHMANN Therapie-Systeme AG, 56626 Andernach (DE)
(72) Erfinder: JUNG, Tobias, 79588 Efringen-Kirchen (DE); HORSTMANN, Michael, 56564 Neuwied (DE); HOFFMANN, Gerd, 56566 Neuwied (DE)
(74) Vertreter: Held, Stephan
(86) Internationale Anmeldenummer: PCT/EP2012/066541
(87) Internationale Veröffentlichungsnummer: WO 2013/030129

(56) Entgegenhaltungen:
- WO-A1-02/05809
- WO-A1-96/06602
- SMITS T ET AL: "New aspects in photodynamic therapy of actinic keratoses", JOURNAL OF PHOTOCHEMISTRY AND PHOTOBIOLOGY B: BIOLOGY, ELSEVIER SCIENCE S.A., BASEL, CH, Bd. 96, Nr. 3, 4. September 2009 (2009-09-04), Seiten 159-169, XP026471323, ISSN: 1011-1344, DOI: 10.1016/J.JPHOTOBIOL.2009.06.003 [gefunden am 2009-06-13]

## Beschreibung

Die vorliegende Erfindung betrifft ein transdermales therapeutisches System bzw. ein transdermales wirkstoffhaltiges Pflaster für 5-Aminolävulinsäurehydrochlorid. Sie betrifft ferner ein derartiges System zur Verwendung in der photodynamischen Diagnostik und Therapie.

Transdermale therapeutische Systeme haben heutzutage als Darreichungsform zur Behandlung zahlreicher Erkrankungen weite Verbreitung gefunden, da sie gegenüber herkömmlichen Verabreichungsformen bestimmte Vorteile zeigen. So können transdermale therapeutische Systeme den therapeutischen Wert eines Wirkstoffes erhöhen, da sie eine konstante Abgabe desselben gewährleisten. Die Vorteile von transdermalen therapeutischen Systemen liegen auch darin, dass sie im Vergleich zu Salben bzw. Cremes flächengenau und damit dosiergenau appliziert werden können. Ferner besteht nicht die Gefahr eines versehentlichen Abwischens der Salbe und der Kontamination anderer Hautstellen.

Aus der EP 1 467 706 A1 ist ein transdermales therapeutisches System zur Freisetzung von 5-Aminolävulinsäure bekannt. 5-Aminolävulinsäure wird selektiv von Tumor-Gewebe aufgenommen und angereichert, wodurch es nur dort zu einer vermehrten Porphyrinbildung und -konzentration führt, während das gesunde Gewebe im Wesentlichen unbeeinflusst bleibt. Die Wirkung der 5-Aminolävulinsäure beruht auf der Stimulierung der körpereigenen Porphyrinbildung. Da das Porphyrin bei Bestrahlung stark fluoresziert, kann die 5-Aminolävulinsäure- bzw. Porphyrin-Anreicherung im erkrankten Gewebe zur Diagnose präkanceröser und kanceröser Läsionen sowie zu deren photodynamischer Therapie ausgenutzt werden. Ein ähnliches System ist auch aus der EP 1 303 267 A1 bekannt. Beide Systeme haben den Nachteil, dass 5-Aminolävulinsäure nur vergleichsweise schlecht durch die menschliche Haut permeatiert. WO 96/06602 offenbart stabile Zubereitungen mit 5-Aminolävulinsäure. Die aktinische Keratose wird als Frühform des weißen Hautkrebses bezeichnet, da diese in 10% der Fälle in einem Zeitraum von 10 Jahren in ein Plattenepithelkarzinom der Haut (Spinaliom) übergehen kann. Sie ist eine durch langjährige intensive Einwirkung von Sonnenlicht (UV-Strahlung) verursachte chronische Schädigung der verhornten Oberhaut. Ein wichtiges Behandlungsverfahren der aktinischen Keratose ist die sogenannte photodynamische Therapie. Hierbei wird zunächst ein Wirkstoff auf die betroffene Hautregion aufgebracht und in den erkrankten Hautzellen bilden sich verstärkt bestimmte lichtempfindliche Substanzen, die sogenannten Porphyrine. Dadurch werden die Zellen für die nachfolgende Behandlung mit Licht sensibilisiert und es entsteht reaktiver Sauerstoff (photodynamischer Effekt), der letztendlich zum Tod der entsprechenden Zellen führt. Mit der photodynamischen Therapie lassen sich meist gute kosmetische Ergebnisse erzielen. Die photodynamische Therapie ist ferner nahezu beliebig oft wiederholbar, wenn die aktinische Keratose erneut auftritt. Neben der therapeutischen Wirkung bietet die photodynamische Therapie außerdem einen diagnostischen Nutzen. Mit speziellem Licht lassen sich die von aktinischer Keratose betroffenen und mit entsprechenden Substanzen vorbehandelten Regionen gezielt sichtbar machen. So ist es möglich, die aktinische Keratose frühzeitig zu erkennen und die Größe der befallenen Stellen genau zu bestimmen (photodynamische Diagnostik).

Aufgabe der vorliegenden Erfindung ist es, ein transdermales therapeutisches System bereitzustellen, welches eine ausreichende Menge eines Stoffes in präkancerösen und kancerösen Läsionen möglichst schnell freisetzt, um anschließend mittels Bestrahlung die photodynamische Therapie durchzuführen. Das transdermale therapeutische System sollte von der Haut gut toleriert werden, flexibel und ausreichend klebrig sein, selbst auf weniger gut zugänglichen Bereichen, wie dem Nasenbein oder der Ohrmuschel. Ferner sollte das transdermale therapeutische System stabil, optisch unauffällig, leicht applizierbar und wieder entfernbar sein.

Obige Aufgabe wird durch ein transdermales therapeutisches System bzw. ein transdermales wirkstoffhaltiges Pflaster gelöst, welches eine wirkstoffundurchlässige Rückschicht, eine wirkstoffhaltige Polymermatrix und eine abziehbare Schutzschicht umfasst, und dadurch gekennzeichnet ist, dass als Wirkstoff 5-Aminolävulinsäurehydrochlorid eingesetzt wird, dass das Basispolymer der Polymermatrix ein haftklebendes Polyacrylat ist und dass das Polyacrylat ohne Vernetzungsmittel erhalten wurde.

Das erfindungsgemäße transdermale therapeutische System mit 5-Aminolävulinsäurehydrochlorid als Wirkstoff und einem haftklebenden Polyacrylat als Basispolymer der Polymermatrix ist in der Lage ausreichend große Mengen des suspendierten Arzneimittels, d. h. des 5-Aminolävulinsäurehydrochlorids, aufzunehmen. Es besteht eine gute Kompatibilität zwischen dem verwendeten haftklebendem Polyacrylat und dem 5-Aminolävulinsäurehydrochlorid. Die Freisetzungsgeschwindigkeit des 5-Aminolävulinsäurehydrochlorids während der Applikationsdauer ist außerordentlich hoch. Ferner haftet das erfindungsgemäße transdermale therapeutische System ausreichend auf der Haut, irritiert diese jedoch nicht. Das erfindungsgemäße transdermale therapeutische System kann leicht appliziert werden, insbesondere auch auf kleinen Hautbereichen, wie der Stirn, der Ohrmuschel oder der Nase.

Das erfindungsgemäße transdermale therapeutische System ist dadurch gekennzeichnet, dass es befähigt ist, innerhalb von etwa vier Stunden, vorzugsweise innerhalb von etwa einer Stunde und besonders bevorzugt innerhalb von etwa 30 Minuten eine Menge von mindestens 3 mg 5-Aminolävulinsäurehydrochlorid freizusetzen (gemessen als 5-Aminolävulinsäure mit dem sogenannten "paddle over disk" Verfahren, wie in European Pharmacopoeia 6.0, 2.9.4. "dissolution test for transdermal patches", 01/2008 : 20904 beschrieben; siehe auch Beispiel 4).

Vorzugsweise handelt es sich bei dem erfindungsgemäßen transdermalen therapeutischen System um ein monolithisches Wirkstoff-in-Klebstoff-System (monolithic drug-in-adhesive system). Hierbei wird 5-Aminolävulinsäurehydrochlorid direkt in die Polymermatrix suspendiert bzw. dispergiert. Die Polymermatrix übt dabei die drei Funktionen des Wirkstoffreservoirs, des Kontrollelements und der Haftschicht aus. Ein derartiges System besteht lediglich aus einer wirkstoffundurchlässigen Rückschicht, einer wirkstoffhaltigen Polymermatrix und einer abziehbaren Schutzschicht. Die Polymermatrix beeinflusst das Haften auf der Haut, die Aufbewahrung des 5-Aminolävulinsäurehydrochlorids und die Freisetzung desselben. Ein derartiges System führt bei der Freisetzung von hydrophilen Substanzen, wie 5-Aminolävulinsäurehydrochlorid, zu mehreren Vorteilen. So können weitere hydrophile Matrixmaterialien vermieden werden, wodurch die mikrobiologische Stabilität verbessert wird. Auch die Stabilität des Wirkstoffs ist erhöht, da dieser chemisch inaktiviert ist. Ferner ist es möglich, die Freisetzung des Wirkstoffes über die Teilchengröße zu steuern.

Die wirkstoffundurchlässige Rückschicht ist vorzugsweise inert und möglichst flexibel, so dass das transdermale therapeutische System auch auf ungleichmäßigen Hautbereichen aufgetragen werden kann. Für die Rückschicht kann jedes geeignete Material, wie zum Beispiel Polyethylenterephthalat, Polyethylen, Polybutylen, Polyurethan, Polyester, etc., verwendet werden. Vorzugsweise handelt es sich bei der wirkstoffundurchlässigen Rückschicht um eine gegebenenfalls aluminisierte Polyesterfolie, besonders bevorzugt um ein Laminat aus pigmentiertem Polyethylen und mit Aluminiumdampf beschichtetem Polyester, welche Schutz vor Lichteinstrahlung und damit Photosensibilisierung vor der eigentlichen photodynamischen Therapie verhindern.

Die abziehbare Schutzschicht kann aus verschiedenen Materialien, wie zum Beispiel Polyethylenterephthalat, Polyethylen oder Polypropylen, hergestellt werden und ist auf der mit der wirkstoffhaltigen Polymermatrix in Kontakt stehenden Seite speziell behandelt, um sie möglichst leicht von dieser ablösbar zu machen. Vorteilhafterweise handelt es sich bei der abziehbaren Schutzschicht um eine Polyethylenterephthalat-Schicht.

In einer bevorzugten Ausführungsform liegt der Wirkstoff 5-Aminolävulinsäurehydrochlorid als kristallines 5-Aminolävulinsäurehydrochlorid vor. Dies hat den Vorteil, dass die Löslichkeit des Wirkstoffes in der Matrix nicht eingestellt werden muss. Ferner wird dadurch eine Übersättigung und ein gleichbleibender Diffusionsdruck erreicht.

In einer bevorzugten Ausführungsform sind etwa 50 % der Kristalle bzw. Teilchen des kristallinen 5-Aminolävulinsäurehydrochlorids größer als die Schichtdicke der Polymermatrix. Der Wirkstoff ragt sozusagen aus der Matrix heraus, was den Vorteil hat, dass sich bei Kontakt mit der Haut, insbesondere mit dem Schweiß, die herausragenden Kristalle sehr schnell auflösen und somit leicht und schnell transdermal aufgenommen werden können.

Vorzugsweise sind mehr als 99,9% der Kristalle des kristallinen 5-Aminolävulinsäurehydrochlorids kleiner als etwa 250 µm. Zwar nimmt bei größer werdender Kristallgröße der epidermale Fluss zu, jedoch führen zu große Kristalle, d. h. Kristallgrößen über etwa 250 µm, zu Verklumpungen und Schlierenbildung.

Andererseits ist es bevorzugt, dass die Menge an Kristallen, die kleiner als 90 µm sind, höchstens 50%, und die Menge an Kristallen, die kleiner als 50 µm sind, höchstens 25%, der Wirkstoffmasse ausmachen, da dies einen hohen Wirkstofffluss sichert.

Ein transdermales therapeutisches System mit 5-Aminolävulinsäurehydrochlorid-Kristallen einer Teilchengröße von 90 bis 160 µm zeigt einen deutlich verbesserten transepidermalen Fluss als ein System mit Teilchen einer Teilchengröße von kleiner als 90 µm. Dies liegt wohl daran, dass mehr Wirkstoff gelöst ist und damit für die Permeation zur Verfügung steht. Teilchengrößen im Bereich von 90 bis 160 µm sind daher besonders bevorzugt.

Vorzugsweise enthält die Polymermatrix des erfindungsgemäßen transdermalen therapeutischen Systems weniger als 30 Gew.-%, bevorzugt weniger als 20 Gew.-%, und besonders bevorzugt weniger als 5 Gew.-% Weichmacher, beispielsweise Citronensäureester, wie Acetyltributylcitrat, bezogen auf Polyacrylat. Ganz besonders bevorzugt liegt der Weichmachergehalt im erfindungsgemäßen transdermalen therapeutischen System unter 5000 ppm.

Vorzugsweise kann auch auf sog. Enhancer bzw. Permeationspromotoren verzichtet werden.

Das haftklebende Polyacrylat wird ohne Vernetzungsmittel (crosslinker), wie beispielsweise Aluminiumacetylacetonat, Polybutyltitanat oder t-Amylperoxypirrolat, etc., erhalten.

Vorzugsweise weist das haftklebende Polyacrylat Säurefunktionalitäten (Carboxylgruppen) auf, da diese in Bezug auf die Adhäsion vorteilhaft sind. Diese sind insbesondere dann wichtig, wenn die Kristalle des kristallinen 5-Aminolävulinsäurehydrochlorids größer als die Schichtdicke der Polymermatrix sind, da dann ein vollflächiger Kontakt zwischen dem transdermalen therapeutischen System und der Haut nicht sichergestellt ist. Das haftklebende Polyacrylat mit Säurefunktionalitäten kann beispielsweise durch Polymerisation eines Monomerengemisches erhalten werden, welches eine ungesättigte Carbonsäure, wie z.B. Acrylsäure, Methacrylsäure oder Maleinsäure, enthält.

Die zur Herstellung der haftklebenden Polyacrylate verwendeten Monomerengemische können auch Acrylsäurederivate mit Epoxygruppen enthalten, wie z.B. Glycidyl(meth)acrylat.

Das Polyacrylat basiert vorzugsweise auf Acrylestern, wie beispielsweise 2-Ethylhexylacrylat. Vorzugsweise wird dieses in einer Menge von mehr als 50 Gew.-%, insbesondere mehr als 60 Gew.-% und besonders bevorzugt in einer Menge von mehr als 70 Gew.-%, bezogen auf Polyacrylat, eingesetzt.

Die Viskosität des Polyacrylats liegt vorzugsweise im Bereich von 500 bis 25000, besonders bevorzugt im Bereich von 1000 bis 20000 und ganz besonders bevorzugt im Bereich von 1500 bis 12000 mPa.s bei 25°C.

In einer bevorzugten Ausführungsform handelt es sich bei dem haftklebenden Polyacrylat um Polyacrylate auf Basis von Acrylsäure, Butylacrylat, 2-Ethylhexylacrylat und Vinylacetat, auf Basis von 2-Ethylhexylacrylat, 2-Hydroxyethylacrylat und Vinylacetat, auf Basis von Acrylsäure, 2-Ethylhexylacrylat und Methylacrylat, auf Basis von Acrylsäure, 2-Ethylhexylacrylat und Vinylacetat, auf Basis von 2-Ethylhexylacrylat, 2-Hydroxyethylacrylat und Methylacrylat, auf Basis von 2-Ethylhexylacrylat, 2-Hydroxyethylacrylat und Methylacrylat, auf Basis von Acrylsäure, Butylacrylat, 2-Ethylhexylacrylat, Vinylacetat, 2-Hydroxyethylacrylat und Methylmethacrylat, auf Basis von Acrylsäure, Butylacrylat, 2-Ethylhexylacrylat, Vinylacetat, t-Octylacrylamid und Vinylacetat und auf Basis von 2-Ethylhexylacrylat und Vinylacetat.

Besonders bevorzugt sind haftklebende Polyacrylate auf Basis von Acrylsäure, Butylacrylat, 2-Ethylhexylacrylat und Vinylacetat sowie haftklebende Polyacrylate auf Basis von Acrylsäure, 2-Ethylhexylacrylat und Methylacrylat, wobei letzteres die besten Ergebnisse liefert.

Das zuerst genannte haftklebende Polyacrylat auf Basis von Acrylsäure, Butylacrylat, 2-Ethylhexylacrylat und Vinylacetat wird vorzugsweise hergestellt aus einem Monomerengemisch, welches 1 - 10 Gew.-%, vorzugsweise 3 - 7 Gew.-%, und besonders bevorzugt etwa 5 Gew.-% Acrylsäure, 5-25 Gew.-%, vorzugsweise 10-20 Gew.-%, und besonders bevorzugt etwa 15 Gew.-% Butylacrylat, 60 - 80 Gew.-%, vorzugsweise 70 - 78 Gew.-%, und besonders bevorzugt etwa 75 Gew.-% 2-Ethylhexylacrylat, und 1 - 10 Gew.-%, vorzugsweise 2 - 8 Gew.-%, und besonders bevorzugt etwa 5 Gew.-% Vinylacetat enthält.

Das haftklebende Polyacrylat auf Basis von Acrylsäure, 2-Ethylhexylacrylat und Methylacrylat wird vorzugsweise hergestellt aus einem Monomerengemisch, welches 1 - 10 Gew.-%, vorzugsweise 2 - 8 Gew.-%, und besonders bevorzugt etwa 5,7 Gew.-% Acrylsäure, 50 - 70 Gew.-%, vorzugsweise 55 - 65 Gew.-%, und besonders bevorzugt etwa 62,2 Gew.-% 2-Ethylhexylacrylat und 20 - 40 Gew.-%, vorzugsweise 30 - 35 Gew.-%, und besonders bevorzugt etwa 32 Gew.-% Methylacrylat enthält. Bei letzterem können auch geringe Mengen Glycidylmethacrylat, beispielsweise kleiner als 1 Gew.-%, vorzugsweise kleiner als 0,05 Gew.-%, und besonders bevorzugt etwa 0,03 Gew.-%, Glycidylmethacrylat vorliegen.

In einer bevorzugten Ausführungsform enthält die Polymermatrix des erfindungsgemäßen transdermalen therapeutischen Systems mehr als 10 Gew.-% und besonders bevorzugt mehr als 20 Gew.-% 5-Aminolävulinsäurehydrochlorid. Es wurde experimentell festgestellt, dass sich die Freisetzung des 5-Aminolävulinsäurehydrochlorids innerhalb der ersten Stunde durch Erhöhung der Wirkstoffmenge von 20 Gew.-% auf 30 Gew.-% etwa um den Faktor 6 erhöht.

Andererseits führen zu große Mengen an 5-Aminolävulinsäurehydrochlorid zu einer Verschlechterung der Haftbarkeit auf der Haut und zu Problemen bei der Beschichtung. Daher ist es bevorzugt, dass weniger als 35 Gew.-% und insbesondere weniger als 30 Gew.-% 5-Aminolävulinsäurehydrochlorid vorliegen. Der Bereich zwischen 25 bis 30 Gew.-% ist optimal.

Die Menge des im erfindungsgemäßen transdermalen therapeutischen Systems eingesetzten Polyacrylats beträgt vorzugsweise mehr als 60 Gew.-% und besonders bevorzugt mehr als 70 Gew.-%.

In einer bevorzugten Ausführungsform ist das transdermale therapeutische System ein monolithisches Wirkstoff-in-Klebstoff-System, mehr als 99,9 % der Kristalle des kristallinen 5-Aminolävulinsäurehydrochlorids sind kleiner als 250 µm, das haftklebende Polyacrylat basiert auf Acrylsäure, Butylacrylat, 2-Ethylhexylacrylat und Vinylacetat und besonders bevorzugt auf Acrylsäure, 2-Ethylhexylacrylat und Methylacrylat, und die Polymermatrix enthält 25 bis 30 Gew.-%, vorzugsweise etwa 28 Gew.-%, 5-Aminolävulinsäurehydrochlorid und 70 oder mehr Gew.-%, vorzugsweise etwa 72 Gew.-%, Polyacrylat. Ein derartiges System zeigt eine sehr schnelle Freisetzung einer großen Wirkstoffmenge und die Verarbeitungsfähigkeit ist hervorragend.

Die Herstellung des erfindungsgemäßen transdermalen therapeutischen Systems erfolgt auf bekannte Art und Weise. Zunächst wird eine wirkstoffhaltige Klebstoffmasse auf Basis eines haftklebenden Polyacrylats hergestellt. Als Lösungsmittel kommen vorzugsweise Ethanol, Ethylacetat, Heptan, Hexan, Isopropylalkohol, Methanol, Toluol, 2,4-Pentandien sowie Gemische davon in Frage. Besonders bevorzugt sind Ethylacetat und Hexan. Es schließen sich übliche Beschichtungs-, Trocknungs- und Laminierungs-Verfahren sowie Schneiden an. Das Lösungsmittel wird bei der Trocknung nahezu vollständig entfernt. Schließlich folgen Lochung und Verpackung.

Die vorliegende Erfindung betrifft auch das erfindungsgemäße transdermale therapeutische System zur Verwendung bei der Diagnose und Therapie von Hautkrebsvorstufen, wie aktinischer Keratose, und von Hautkrebs bzw. onkologischen Hauterkrankungen. Die äußerliche Applikation des transdermalen therapeutischen Systems führt zur Penetration und Anreicherung des Wirkstoffes im erkrankten Gewebe. 5-Aminolävulinsäurehydrochlorid ist eine endogene Verbindung und eine Vorläufersubstanz in der Biosynthese von Porphyrinen, die Bestandteile beispielsweise des Hämoglobin- und des Cytochromzyklus sind. 5-Aminolävulinsäure-hydrochlorid wird zum eigentlichen Photosensibilisator, dem Protoporphyrin IX (PPIX), umgewandelt. Nach der Anreicherung erfolgt eine Bestrahlung mit adäquatem Licht, beispielsweise mit Licht verschiedener Wellenlängen, wie zum Beispiel 408 mm, 506 mm, 532 mm, 580 mm und 635 mm. Dabei entstehen reaktive Sauerstoffverbindungen, welche das Zielgewebe bei der Diagnose sichtbar bzw. bei der Therapie zu einer Apoptose und Nekrose desselben führen.

Die vorliegende Erfindung betrifft auch ein transdermales therapeutisches System, wie vorstehend beschrieben, zur Verwendung als therapeutisches Mittel.

Ferner ist ein transdermales therapeutisches System, wie vorstehend beschrieben, zur Behandlung von Hautkrebsvorstufen, wie beispielsweise aktinischer Keratose, und onkologischen Hauterkrankungen, offenbart. Das transdermale therapeutische System kann zur Behandlung von aktinischer Keratose eingesetzt werden.

### Beispiele

### Beispiel 1

Das hergestellte transdermale therapeutische System enthält folgende Bestandteile:

| | | % (Gew.-%/Gew.-%) | mg/Pflaster |
|---|---|---|---|
| Wirkstoff: | 5-Aminolävulinsäurehydrochlorid | 19,7 | 10,2 |
| Polymer ^{a)}: | DURO-TAK 387-2353 | 49,6 | 25,8 |
| Rückschicht: | 3 M ScotchPak 1109 | 30,7 | 16,0 |
| Schutzschicht: | Einseitig silikonisierte Polyethylenterephthalat-Schicht (75 µm) | ^{b)} | 59,0 ^{c)} |

| | | | |
|---|---|---|---|
| ^{a)} in Ethylacetat und Hexan, die beide während der Trocknung nahezu vollständig entfernt werden ^{b)} wird vor der Applikation entfernt ^{c)} geschätzt | | | |

Bezogen auf die Polymermatrix liegen demnach 28 Gew.-% 5-Aminolävulinsäurehydrochlorid und 72 Gew.-% DURO-TAK 387-2353 (Polyacrylat ohne Vernetzungsmittel) vor.

### Beispiel 2 (nicht erfindungsgemäß)

Die Zusammensetzung dieses Beispiels entspricht Beispiel 1, außer dass anstelle von DURO-TAK 387-2353 die gleiche Menge DURO-TAK 387-2052 (Polyacrylat mit Vernetzungsmittel) eingesetzt wurde.

### Vergleichsbeispiel 3

Die Zusammensetzung dieses Beispiels entspricht Beispiel 1, außer dass anstelle von DURO-TAK 387-2353 die gleiche Menge Bio-PSA 4301 (ein Silikonpolymer) eingesetzt wurde.

### Beispiel 4

Die Freisetzungsgeschwindigkeit wurde mit dem sogenannten "paddle over disk" Verfahren, wie in European Pharmacopoeia 6.0, 2.9.4. "dissolution test for transdermal patches", 01/2008 : 20904 beschrieben, unter folgenden Bedingungen gemessen:

| | |
|---|---|
| Verwendete Apparatur: | Paddle over disk |
| Freisetzungsmedium: | Citratpuffer pH 3,0 |
| Volumen des Freisetzungsmediums: | 300 ml |
| Temperatur: | 32°C ± 0.5°C |
| Rotationsfrequenz: | 50 min ⁻¹ |
| Probenentnahme-Zeitpunkt: | 0,5 h, 2 h und 7 h |
| Probenvolumen: | 10,0 ml |

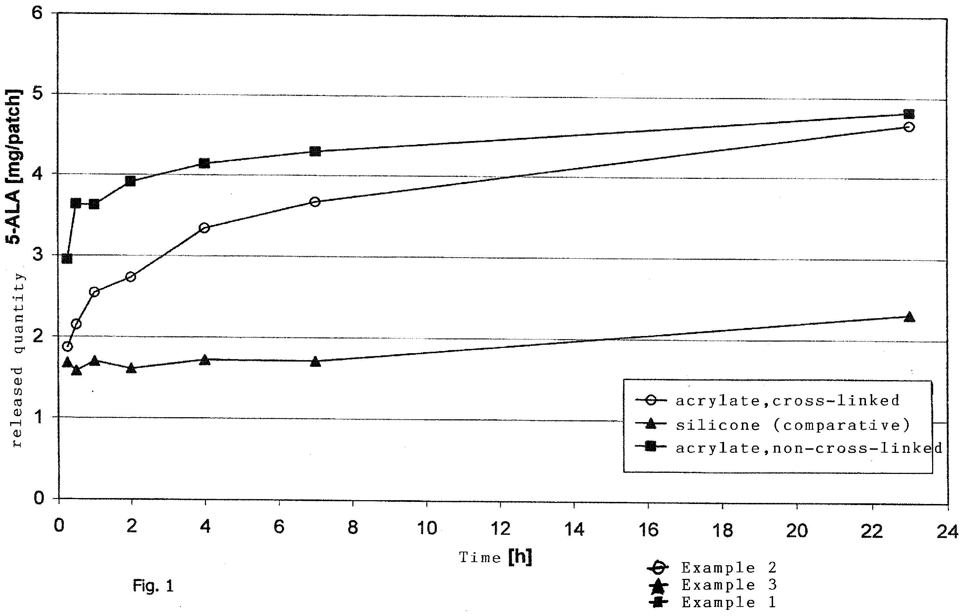

Die Ergebnisse sind in Fig. 1 gezeigt.

Die Freisetzungsgeschwindigkeit eines transdermalen therapeutischen Systems gemäß Beispiel 1 ist höher als diejenige gemäß Beispiel 2. Sowohl Beispiel 1 als auch Beispiel 2 zeigen eine deutlich schnellere Freisetzung verglichen mit Vergleichsbeispiel 3. Ferner ist die Stabilität des 5-Aminolävulinsäurehydrochlorids gemäß Beispiel 1 nach einem und drei Monaten höher als die des 5-Aminolävulinsäurehydrochlorids gemäß Beispiel 2, welches schneller abgebaut wird.

## Patentansprüche

1. Transdermales therapeutisches System, umfassend eine wirkstoffundurchlässige Rückschicht, eine wirkstoffhaltige Polymermatrix und eine abziehbare Schutzschicht, **dadurch gekennzeichnet, dass** der Wirkstoff 5-Aminolävulinsäurehydrochlorid ist, dass das Basispolymer der Polymermatrix ein haftklebendes Polyacrylat ist und dass das Polyacrylat ohne Vernetzungsmittel erhalten wurde.

2. Transdermales therapeutischen System nach Anspruch 1, **dadurch gekennzeichnet, dass** das transdermale therapeutische System ein monolithisches Wirkstoff-in-Klebstoff-System ist.

3. Transdermales therapeutisches System nach mindestens einem der vorangegangenen Ansprüche, **dadurch gekennzeichnet, dass** das 5-Aminolävulinsäurehydrochlorid ein kristallines 5-Aminolävulinsäurehydrochlorid ist.

4. Transdermales therapeutisches System nach mindestens einem der vorangegangenen Ansprüche, **dadurch gekennzeichnet, dass** 50 % der Kristalle des kristallinen 5-Aminolävulinsäurehydrochlorid größer als die Schichtdicke der Polymermatrix sind.

5. Transdermales therapeutisches System nach mindestens einem der vorangegangenen Ansprüche, **dadurch gekennzeichnet, dass** mehr als 99,9% der Kristalle des kristallinen 5-Aminolävulinsäurehydrochlorid kleiner als etwa 250 µm sind und besonders bevorzugt eine Teilchengröße von 90 bis 160 µm haben.

6. Transdermales therapeutisches System nach mindestens einem der vorangegangenen Ansprüche, **dadurch gekennzeichnet, dass** die Polymermatrix weniger als 30 Gew.-%, bevorzugt weniger als 20 Gew.-% und besonders bevorzugt weniger als 5 Gew.-% Weichmacher, bezogen auf Polyacrylat, enthält.

7. Transdermales therapeutisches System nach mindestens einem der vorangegangenen Ansprüche, **dadurch gekennzeichnet, dass** das Polyacrylat Säurefunktionalitäten aufweist.

8. Transdermales therapeutisches System nach mindestens einem der vorangegangenen Ansprüche, **dadurch gekennzeichnet, dass** das Polyacrylat ein Polyacrylat auf Basis von Acrylsäure, Butylacrylat, 2-Ethylhexylacrylat und Vinylacetat oder auf Basis von Acrylsäure, 2-Ethylhexylacrylat und Methylacrylat, ist.

9. Transdermales therapeutisches System nach mindestens einem der vorangegangenen Ansprüche, **dadurch gekennzeichnet, dass** die Polymermatrix mehr als 10 Gew.-%, bevorzugt mehr als 20 Gew.-% und besonders bevorzugt 25 bis 30 Gew.-% 5-Aminolävulinsäurehydrochlorid enthält.

10. Transdermales therapeutisches System nach mindestens einem der vorangegangenen Ansprüche, **dadurch gekennzeichnet, dass** die Polymermatrix mehr als 60 Gew.-% Polyacrylat, bevorzugt mehr als 65 Gew.-% und besonders bevorzugt mehr als 70 Gew.-% Polyacrylat enthält.

11. Transdermales therapeutisches System nach mindestens einem der vorangegangenen Ansprüche, **dadurch gekennzeichnet, dass** das transdermale therapeutische System ein monolithisches Wirkstoff-in-Klebstoff-System ist und das die Polymermatrix etwa 28 Gew.-% kristallines 5-Aminolävulinsäurehydrochlorid und etwa 72 Gew.-% Polyacrylat auf Basis von Acrylsäure, 2-Ethylhexylacrylat und Methylacrylat, welches ohne Vernetzungsmittel erhalten wurde, enthält.

12. Transdermales therapeutisches System nach mindestens einem der vorangegangenen Ansprüche zur Verwendung als therapeutisches Mittel.

13. Transdermales therapeutisches System nach einem der Ansprüche 1 bis 12 zur Verwendung bei der Diagnose und Behandlung von Hautkrebsvorstufen, wie beispielsweise aktinischer Keratose, und onkologischen Hauterkrankungen.

## Claims

1. A transdermal therapeutic system including an active substance-impermeable rear layer, an active substance-containing polymer matrix and a peelable protective layer, **characterised in that** the active substance is 5-aminolevulinic acid hydrochloride, that the base polymer of the polymer matrix is a self-adhesive polyacrylate and that the polyacrylate was produced without a cross-linking agent.

2. A transdermal therapeutic system as claimed in Claim 1, **characterised in that** the transdermal therapeutic system is a monolithic active substance-in-adhesive system.

3. A transdermal therapeutic system as claimed in at least one of the preceding claims, **characterised in that** the 5-aminolevulinic acid hydrochloride is a crystalline 5-aminolevulinic acid hydrochloride.

4. A transdermal therapeutic system as claimed in at least one of the preceding claims, **characterised in that** 50% of the crystals of the crystalline 5-aminolevulinic acid hydrochloride are larger than the layer thickness of the polymer matrix.

5. A transdermal therapeutic system as claimed in at least one of the preceding claims, **characterised in that** more than 99.9% of the crystals of the crystalline 5-aminolevulinic acid hydrochloride are smaller than about 250 µm and, particularly preferably, have a particle size of 90 to 160 µm.

6. A transdermal therapeutic system as claimed in at least one of the preceding claims, **characterised in that** the polymer matrix contains less than 30 wt.%, preferably less than 20 wt.% and particularly preferably less than 5 wt.% plasticizer, with respect to polyacrylate.

7. A transdermal therapeutic system as claimed in at least one of the preceding claims, **characterised in that** the polyacrylate has acid functionalities.

8. A transdermal therapeutic system as claimed in at least one of the preceding claims, **characterised in that** the polyacrylate is a polyacrylate on the basis of acrylic acid, butylacrylate, 2-ethylhexylacrylate and vinyl acetate or on the basis of acrylic acid, 2-ethylhexylacrylate and methylacrylate.

9. A transdermal therapeutic system as claimed in at least one of the preceding claims, **characterised in that** the polymer matrix includes more than 10 wt.%, preferably more than 20 wt.% and particularly preferably 25 to 30 wt.% 5-aminolevulinic acid hydrochloride.

10. A transdermal therapeutic system as claimed in at least one of the preceding claims, **characterised in that** the polymer matrix includes more than 60 wt.% polyacrylate, preferably more than 65 wt.% and particularly preferably more than 70 wt.% polyacrylate.

11. A transdermal therapeutic system as claimed in at least one of the preceding claims, **characterised in that** the transdermal therapeutic system is a monolithic active substance-in-adhesive system and that the polymer matrix includes about 28 wt.% crystalline 5-aminolevulinic acid hydrochloride and about 72 wt.% polyacrylate on the basis of acrylic acid, 2-ethylhexylacrylate and methylacrylate, which was obtained without crosslinking agent.

12. A transdermal therapeutic system as claimed in at least one of the preceding claims for use as a therapeutic agent.

13. A transdermal therapeutic system as claimed in one of Claims 1 to 12 for use in the diagnosis and treatment of preliminary stage skin cancers, such as actinic keratosis and oncological skin diseases.

## Revendications

1. Système thérapeutique transdermique, comprenant une couche arrière imperméable à la substance active, une matrice polymère contenant la substance active et une couche protectrice amovible, **caractérisé en ce que** la substance active est le chlorhydrate d'acide 5-aminolévulinique, **en ce que** le polymère de base de la matrice polymère est un polyacrylate adhésif et **en ce que** le polyacrylate a été obtenu sans agent de réticulation.

2. Système thérapeutique transdermique selon la revendication 1, le système thérapeutique transdermique étant **caractérisé en ce qu'**il est un système monolithique substance active-dans-adhésif.

3. Système thérapeutique transdermique selon au moins l'une des revendications précédentes, **caractérisé en ce que** le chlorhydrate d'acide 5-aminolévulinique est un chlorhydrate d'acide 5-aminolévulinique cristallin.

4. Système thérapeutique transdermique selon au moins l'une des revendications précédentes, **caractérisé en ce que** 50 % des cristaux du chlorhydrate d'acide 5-aminolévulinique cristallin sont supérieurs à l'épaisseur de couche de la matrice polymère.

5. Système thérapeutique transdermique selon au moins l'une des revendications précédentes, **caractérisé en ce que** plus de 99,9 % des cristaux du chlorhydrate d'acide 5-aminolévulinique cristallin sont inférieurs à environ 250 µm et ont particulièrement de préférence une taille de particules de 90 à 160 µm.

6. Système thérapeutique transdermique selon au moins l'une des revendications précédentes, **caractérisé en ce que** la matrice polymère contient moins de 30 % en poids, de préférence moins de 20 % en poids et particulièrement de préférence moins de 5 % en poids de plastifiant, par rapport au polyacrylate.

7. Système thérapeutique transdermique selon au moins l'une des revendications précédentes, **caractérisé en ce que** le polyacrylate présente des fonctionnalités acides.

8. Système thérapeutique transdermique selon au moins l'une des revendications précédentes, **caractérisé en ce que** le polyacrylate est un polyacrylate à base d'acide acrylique, d'acrylate de butyle, d'acrylate de 2-éthyl-hexyle et d'acétate de vinyle ou à base d'acide acrylique, d'acrylate de 2-éthylhexyle et d'acrylate de méthyle.

9. Système thérapeutique transdermique selon au moins l'une des revendications précédentes, **caractérisé en ce que** la matrice polymère contient plus de 10 % en poids, de préférence plus de 20 % en poids et particulièrement de préférence 25 à 30 % en poids de chlorhydrate d'acide 5-aminolévulinique.

10. Système thérapeutique transdermique selon au moins l'une des revendications précédentes, **caractérisé en ce que** la matrice polymère contient plus de 60 % en poids de polyacrylate, de préférence plus de 65 % en poids et particulièrement de préférence plus de 70 % en poids de polyacrylate.

11. Système thérapeutique transdermique selon au moins l'une des revendications précédentes, le système thérapeutique transdermique étant **caractérisé en ce qu'**il est un système monolithique substance active-dans-adhésif et **en ce que** la matrice polymère contient environ 28 % en poids de chlorhydrate d'acide 5-aminolévulinique cristallin et environ 72 % en poids de polyacrylate à base d'acide acrylique, d'acrylate de 2-éthylhexyle et d'acrylate de méthyle, qui a été obtenu sans agent de réticulation.

12. Système thérapeutique transdermique selon au moins l'une des revendications précédentes pour une utilisation comme agent thérapeutique.

13. Système thérapeutique transdermique selon l'une des revendications 1 à 12 destiné à être utilisé au cours du diagnostic et du traitement des stades précurseurs du cancer de la peau, comme par exemple la kératose actinique, et des affections dermatologiques oncologiques.
